# EUROPEAN PATENT SPECIFICATION

(11) **EP 0 453 602 B1**
(45) Date of publication and mention of the grant of the patent: **05.07.1995**
(21) Application number: 90107936.8
(22) Date of filing: 26.04.1990
(51) Int. Cl.: A61N 2/02

(54) **Magnetic field generating therapeutical apparatus**
Therapeutische Einrichtung zur Erzeugung magnetischer Felder
Appareil thérapeutique générant des champs magnétiques

(43) Date of publication of application: 30.10.1991
(73) Proprietor: NIHONKENKOZOSHINKENKYUKAI CO. LTD., Fukuoka-shi Fukuoka 813 (JP)
(72) Inventor: Masuda, Isamu, Chuo-ku, Fukuoka-shi, Fukuoka 814-01 (JP)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 356 514
- FR-A- 646 314
- US-A- 2 777 099
- US-A- 4 162 471

## Description

### TECHNICAL FIELD OF THE INVENTION

The present invention relates to a magnetic field generating therapeutical apparatus according to the preamble of claim 1 for applying the magnetic field to the physical portions of patients having suffering of stiffness, pain or the like by said apparatus.

### TECHNICAL BACKGROUND OF THE INVENTION

A conventional magnetic field generating therapeutical apparatus of this kind is disclosed in EP-A-0 356 541 as shown in Fig. 1, and includes a plastic case, housing magnetic field generators connected in multiplicity rotatably with a hinge mechanism. The therapeutical apparatus is applied to the patient's physical portion having sufferings of stiffness or pain for applying the alternating magnetic field generated by the magnetic field generator.

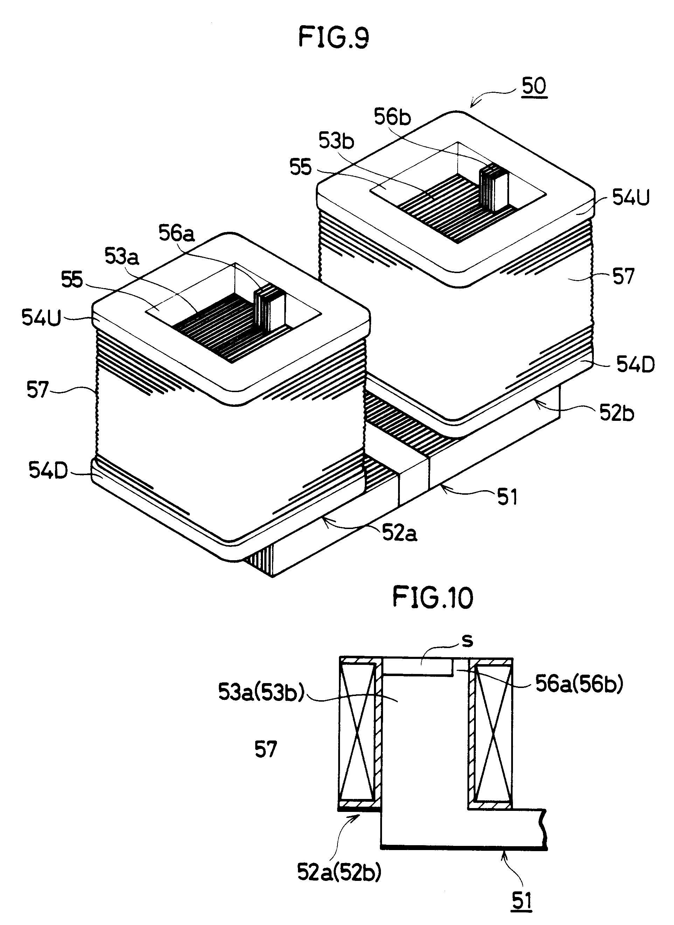

Fig. 9 shows a concrete example of a magnetic field generator 50 as it is contained in case 1 of the known apparatus.

The magnetic field generator 50 is composed of a laminated iron core 51 and a pair of coil bobbins 52a, 52b, being formed by fitting on the coil bobbin respectively on each of the leg part 53a, 53b of the iron core 51. Each of coil bobbin 52a, 52b is formed by winding coil 57 around the periphery of the spool having a collar 54u, 54p at both ends. The laminated iron core 51 is integrally provided with a rectangular protuberance 56a, 56b as its part at the tip of its leg part 53a, 53b for the purpose of concentrating the alternating magnetic field that is generated on these protuberances 56a, 56b.

In such a magnetic field generating therapeutical apparatus, when the alternating current is charged on the coil 57, appropriate vibration and heat are obtained besides that the magnetic field is generated from the magnetic field generator, resulting to give forth therapeutic effect of magnetism in addition to massaging and thermal effect to the body.

Then, if any excessive heat should be generated by the magnetic field generator 50, the heat may be transmitted to the patient's body through the case, causing a burn to the body.

The inventor has investigated, therefore, the reason for the generation of this sort of excessive heat by the magnetic field generator 50 and found that such excessive heat is generated when there is an empty space S inside the coil 57 in fitting the coil bobbins 52a, 52b on the leg part 53a, 53b of the laminated iron core 51 as shown in Fig. 10.

### TECHNICAL PROBLEM

The present invention has been invented in order to offer an effective magnetic field therapeutical apparatus with aforesaid problem kept in mind to provide efficient thermal effect but avoiding the generation of any excessive heat for securing safety for the patient's body.

This object is solved by the features of the characterizing part of claim 1.

In the present invention, in order to obtain on effective thermal effect, aforesaid case is made with far-infrared radioactive substance blended in the molding resin or coated on the case.

Since one side of the collar of the spool wound with coil is formed having an identical height with the protuberance of the laminated iron core so that this collar part comes in a corresponding position of the protuberance and the coil bobbin is fit in on the laminated iron core, no empty space will take place at the inside of coil, restricting the loss of energy to minimum. For this reason, no excessive heat is assured not to entertain any fear of a burn on the patient's body.

Blending far-infrared radioactive substance in molding resin of the case or coating it on the case which excites far-infrared radioactive substance by the heat generated the magnetic field generator, it warms an efficient thermal effect.

### BRIEF DESCRIPTION OF THE DRAWINGS

Fig. 1 is a plan view showing one embodiment of the entire configuration of the magnetic field generating therapeutical apparatus by the present invention.

Fig. 2 is a sectional view along the line II-II in Fig. 1.

Fig. 3 is a plan view showing the interior of one half body of the case.

Fig. 4 is a perspective view showing the shape of the silicon steel plate to be laminated.

Fig. 5 is a perspective view showing the construction of the laminated iron core.

Fig. 6 is a perspective view showing the configuration of the magnetic field generator.

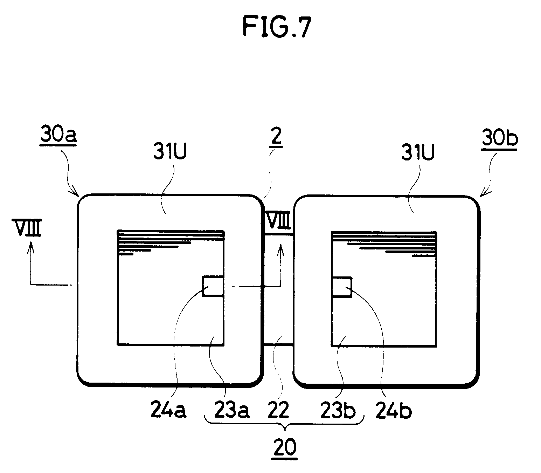

Fig. 7 is a plan view of the magnetic field generator.

Fig. 8 is a sectional view along the line VIII-VIII in Fig. 7.

Fig. 9 is a perspective view of a conventional magnetic field generator.

Fig. 10 is a sectional view of a conventional magnetic field generator.

### PREFERRED EMBODIMENTS

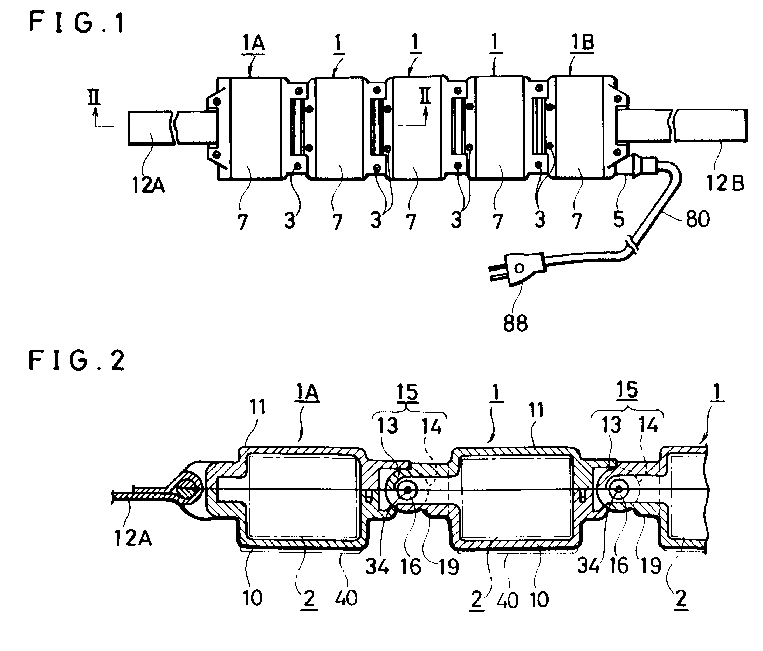

Figs. 1 and 2 show the magnetic field generating therapeutical apparatus in accordance with an embodiment of the present invention, which arranges 2 units of magnetic field generators respectively in one of a multiplicity of plastics-molded cases 1. Each case rotatable is connected with another in freely manner, with two cases - cases A and B located at both ends - are tied with a mutually connectible belt 12A, 12B. After this magnetic field generating therapeutic apparatus is attached onto the part of the patient's body suffering from the stiffness, pain and the like with its side radiating magnetic field (the front half side 10 of the case which will be described later), the magnetic field and the warmth are applied by conducting the electricity.

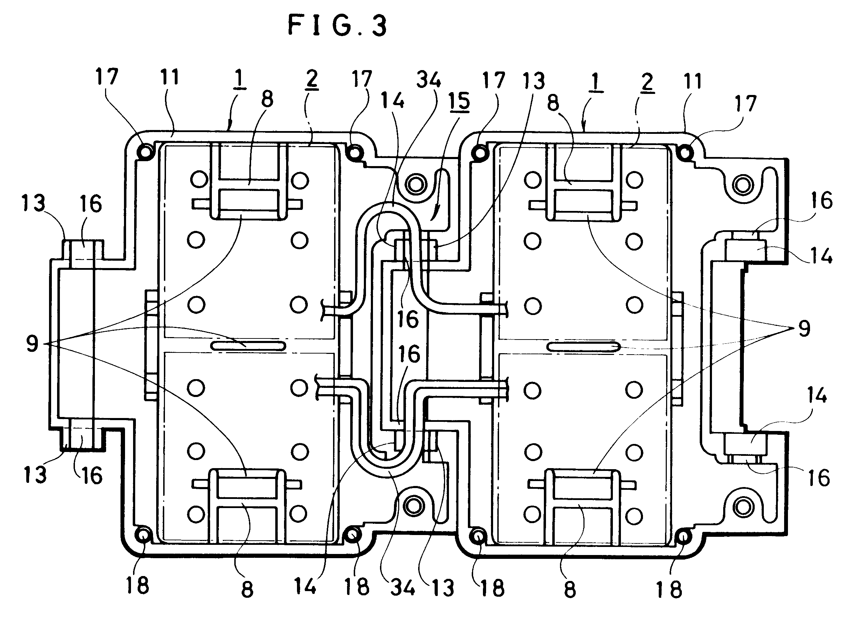

Each case is composed of a front half side 10 paired with the rear half side 11 of the case, with each open side being butted against each other and fastened with a multiplicity of screws 3. At one side end of each case a protuberance 13, 13 is provided in vertical direction, as shown in Figs. 2 and 3, and at the other side end of the case a bearing hole 14, 14 is provided, into which each one protuberance 13 is inserted to form a hinge mechanism 15, making both parts connected in freely rotatable manner to each other.

This hinge mechanism 15 is provided with a through guide hole 16, 16, which penetrates providing the space to a lead wire cord 34 for electrically connecting the magnetic field generator 2, 2 of connecting cases 1, 1.

A case 1 being formed by butting each other of a pair protuberance 13 and bearing hole 14 are divided on the axial center line. At an appropriate, butting surface of the half body 10, 11 a protuberance and a cavity are formed, said protuberance 17 and cavity 18 are set in by butting said two halves against each other. In Fig. 1, numeral 4 is an electric cord, at one end of which a socket 5 and at the other end a plug 6 are provided. In Fig. 2, numeral 19 is a stopper restricting the rotation angle between the cases 1, 1, being formed at the connecting part of each case.

When each case 1 is rotated with the hinge mechanism 15, there is no possibility of the lead wire cord 34 being cut by rubbing or bent as in conventional apparatus since said cord is placed in the guide hole through the rotation center line.

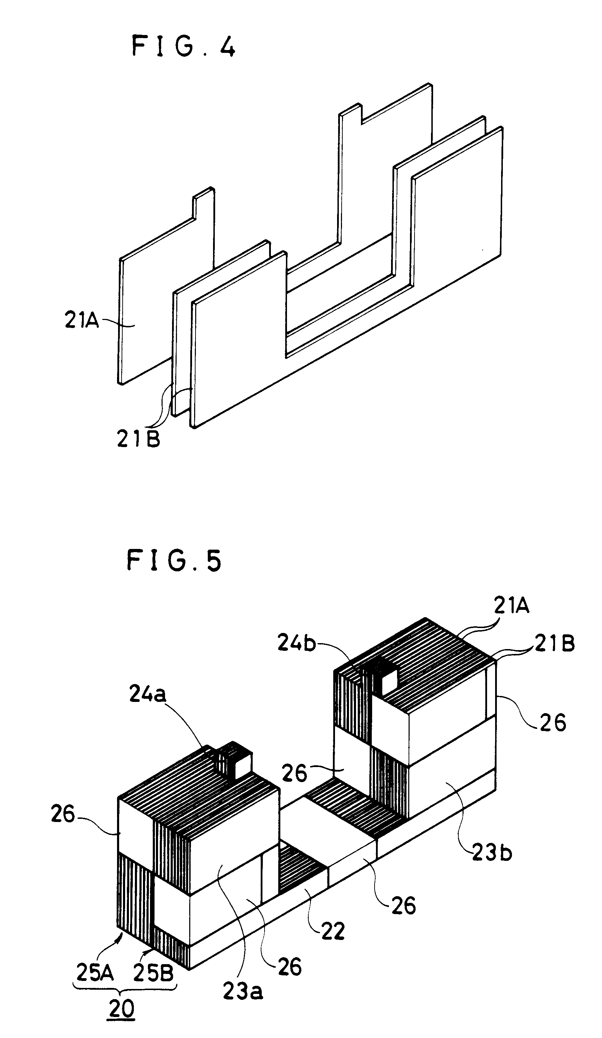

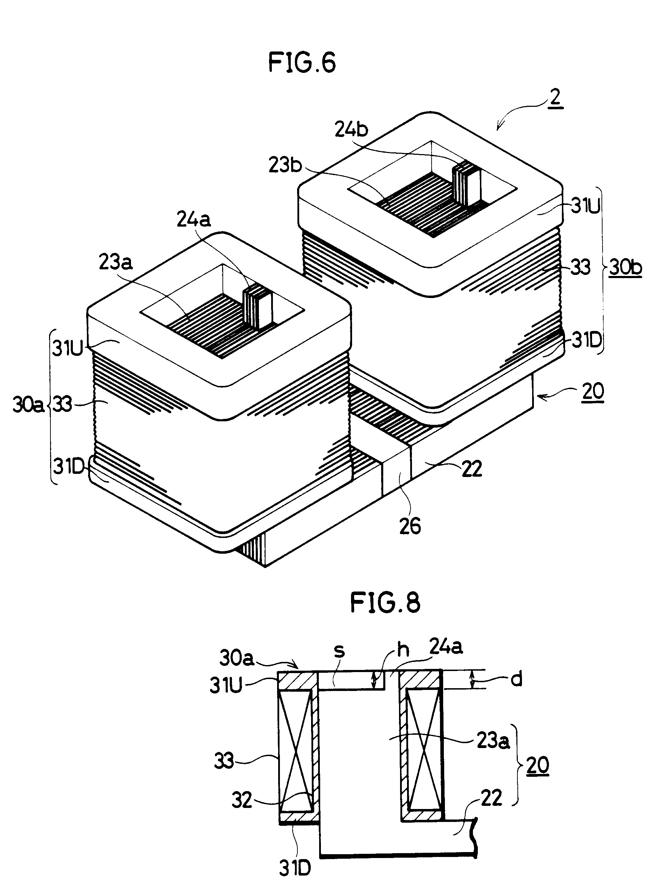

As shown in Fig. 6, the magnetic field generator 2 generated alternating magnetic field by conducting electricity to the coil 33 being arranged on the laminated iron core 20. As shown in Figs. 4 and 5, said laminated iron core 20 is of the structure in which a multiplicity of silicon steel plates 21A, 21B are piled up to an appropriate thickness, around which an isolation tape of heat resisting property 26 are wound. The iron core 20 described in the drawing has a protuberance 24a, 24b for the purpose of concentrating the magnetic fluxed and is composed of a pair of laminated plates 25 having been fixed with a tape as shown in Fig. 5. With such structure of iron cores like this, vibration is stronger compared with the kind of core having the structure of silicon steel plates caulked for fixing.

The iron core 20 assembled in this manner has the leg part 23a, 23b at both ends of its bass 22, and said leg part 23a, 23b are fit with coil bobbin 30a, 30b. Said coil bobbin 30a, 30b is composed of a spool 32 wound with coil 30, with a collar 31u, 31d provided at both ends.

One of said collar 31u of the spool 32 is so made to have an identical height with the protuberance 24a, 24b of the laminated iron core 20, and each coil bobbin 30a, 30b are set on each leg part 23a, 23b so that said collar 31u comes to a position corresponding to said protuberance 24a, 24b. Accordingly, at inside of coil 33, leg part 23a, 23b are located correspondingly, with an empty space S is situated in opposite inside location of the collar 31u.

Aforesaid magnetic field generator 2 is set at its determined position with the position determining piece 8, with the laminated iron core 20 being supported on the protuberance 9 provided at the inside of said half body 11. Furthermore, the magnetic field generator 2 is fixed its position in a state when said collar part 31u of the coil bobbin 30a, 30b is butted against the internal surface of the half body 10 of the case. If the collar part 31u is butted in this way, it will reinforce the magnetic field applying surface 7 of the case's half body 10 from within.

The surface applying the magnetic filed of the case's front half body 10, is coated with same far-infrared radioactive substance such as, for instance, Zirconia, Zircon, titania, Almina, Cordierite or Silica to form a radioactive film 40. Said far-infrared radioactive substance is excited by heat to radiate far-infrared rays. Besides coating some far-infrared radioactive substance on the surface of the case 1, it is possible to blended such substance in the raw material for molding the case 1.

### CONCEIVABLE INDUSTRIAL APPLICATION

When using the magnetic field generating therapeutical apparatus, first, one or a multiplicity of case 1, is fastened and fixed to the part of the patient's body. Then, when a power is applied, each coil 33 of each magnetic field generator 2 is energized and their superposed magnetic fluxes are produced from the end surface of legs 22a, 23b of the laminated iron core 20. Thereby, heat is generated at the iron core 20 and each coil 33, which is conducted to the case body 1. In this case, one side of the collar 31u of the spool 32 being formed identical in height with the protuberance 24a, 24b radioactive of the laminated iron core, and the collar 31u being positioned at the corresponding location of the protuberance 24a, 24b any empty space will not taken place at the inside of the coil 33, restricting the loss of energy to minimum and precluding excessive heat generation.

When an appropriate heat conduction to case 1 takes place, the far-infrared radioactive substance is excited by heating case 1, and far-infrared rays are radiated from the radioactive film 40 of the magnetic field acting surface 7. This will worm the patient's physical interior, promoting the therapeutical effect.

## Claims

1. A magnetic field generating therapeutical apparatus, said apparatus comprising a case (1) containing a magnetic field generator (2) composed of a laminated iron core (20) and a coil bobbin (30a, 30b), wherein aforesaid laminated iron core (20) has an integrally formed protuberance (24a, 24b) extending beyond its end part for concentrating magnetic fluxes and said coil bobbin (30a, 30b) is wound with a coil (33) around a spool (32) having collars (31D, 31U) at both ends,
characterized in that
one of the collars (31U) of said spool (32) is formed to have an identical height with said protuberance (24a, 24b) of the laminated iron core (20), so that the end of the coil (33) of the coil bobbin (30a, 30b) lies in the plane of said end part of the laminated iron core (20) and the free end of said protuberance (24a, 24b) lies flush with the end surface of said one of the collars (31U).

2. A magnetic field generating therapeutical apparatus in accordance with claim 1, wherein afore said case (1) is molded with a far-infrared radiating substance blended or is coated with it.

## Patentansprüche

1. Eine Magnetfeld erzeugende therapeutische Vorrichtung, wobei die Vorrichtung ein Gehäuse (1) umfaßt, das einen Magnetfeldgenerator (2) enthält, der aus einem laminierten Eisenkern (20) und einem Wicklungs-Spulenkörper (30a, 30b) zusammengesetzt ist, worin der vorgenannte laminierte Eisenkern (20) einen einstückig gebildeten Vorsprung (24a, 24b) aufweist, der sich über seinen Endteil zum Konzentrieren des Magnetflusses erstreckt, und der Wicklungsspulenkörper (30a, 30b) mit einer Wicklung (33) um eine Spule (32) gewickelt ist, die Kragen (31D, 31U) bei beiden Enden aufweist,
dadurch **gekennzeichnet,**
daß einer der Kragen (31U) der Spule (32) dazu gebildet ist, eine identische Höhe mit dem Vorsprung (24a, 24b) des laminierten Eisenkernes (20) aufzuweisen, so daß das Ende der Wicklung (33) des Wicklungsspulenkörpers (30a, 30b) in der Ebene des Endteiles des laminierten Eisenkernes (20) liegt, und das freie Ende des Vorsprungs (24a, 24b) bündig mit der Endoberfläche des einen der Kragen (31U) liegt.

2. Eine Magnetfeld erzeugende therapeutische Vorrichtung in Übereinstimmung mit Anspruch 1, worin das vorgenannte Gehäuse (1) mit einer ferninfrarot abstrahlenden Substanz geformt, vermischt oder damit überzogen ist.

## Revendications

1. Appareil thérapeutique qui produit un champ magnétique, ledit appareil comprenant un boîtier (1) qui contient un générateur (2) de champ magnétique, composé d'un noyau de fer en lamelles (20) et d'un bobinage (30a, 30b), dans lequel ledit noyau de fer en lamelles (20) précité comporte une protubérance formée de façon intégrée (24a, 24b) qui s'étend au-delà de sa partie d'extrémité afin de concentrer le flux magnétique, et ledit bobinage (30a, 30b) est enroulé avec une bobine (33) autour d'un tambour (32) qui comporte des colliers (31D, 31U) aux deux extrémités,
caractérisé en ce que l'un des colliers (31U) dudit tambour (32) est formé de façon à présenter une hauteur identique à ladite protubérance (24a, 24b) du noyau de fer en lamelles (20), de sorte que l'extrémité de la bobine (33) du bobinage (30a, 30b) est située dans le plan de ladite partie d'extrémité du noyau de fer (20) en lamelles, et l'extrémité libre de ladite protubérance (24a, 24b) affleure avec la surface terminale dudit collier (31U).

2. Appareil thérapeutique qui produit un champ magnétique, selon la revendication 1, dans lequel ledit boîtier précité (1) est moulé avec un mélange qui contient une substance qui rayonne des rayons infrarouges lointains, ou est revêtu d'une telle substance.
